# EUROPEAN PATENT SPECIFICATION

(11) **EP 3 232 949 B1**
(45) Date of publication and mention of the grant of the patent: **27.01.2021**
(21) Application number: 15868780.6
(22) Date of filing: 15.12.2015
(51) Int. Cl.: A61B 17/132

(54) **SYSTEM FOR ACHIEVING PATENT HEMOSTASIS IN ARTERIES**
SYSTEM ZUR ERZIELUNG VON HÄMOSTASE IN ARTERIEN BEI EINEM PATIENTEN
SYSTÈME PERMETTANT DE PRATIQUER UNE HÉMOSTASE DANS LES ARTÈRES

(30) Priority: 18.12.2014 US 201414575141
(43) Date of publication of application: 25.10.2017
(73) Proprietor: University Health Network (UHN), Toronto, Ontario M5G 2C4 (CA)
(72) Inventor: SHAH, Ashish H., Toronto, Ontario M5G 0B2 (CA)
(74) Representative: Samson & Partner Patentanwälte mbB
(86) International application number: PCT/CA2015/051329
(87) International publication number: WO 2016/095038

(56) References cited:
- EP-A2- 1 382 306
- CN-U- 203 988 215
- JP-A- 2004 041 599
- JP-A- 2005 318 998
- JP-A- 2007 075 650
- US-A- 5 413 582
- US-A- 5 728 134
- US-A1- 2008 269 659
- US-A1- 2010 179 586
- US-A1- 2010 179 586
- US-A1- 2013 237 866
- US-A1- 2015 018 869
- US-B1- 6 264 673

## Description

### BACKGROUND OF THE INVENTION

The field of the invention is systems for providing patent hemostasis in arteries after an interventional procedure, such as angioplasty, guided by a medical imaging technique, such as angiography. More particularly, the invention relates to systems and methods for providing occlusive pressure to one artery in a patient's wrist (e.g., the ulnar artery or the radial artery) and patent hemostasis to another artery in the patient's wrist (e.g., the radial artery or the ulnar artery) using a vascular hemostatic device that is configured to simultaneously compress both the ulnar and radial arteries.

When a procedure involving the percutaneous insertion of an instrument, such as a catheter into a blood vessel, is carried out for medical treatment, examination or diagnosis, bleeding at the puncture site following subsequent withdrawal and removal of the catheter must be stopped. Hemostatic devices that are attached by being wrapped around the portion of an arm where the puncture site is located, thereby compressing the puncture site where bleeding is to be stopped, are known. However, many conventional hemostatic devices are configured to stop bleeding at the puncture site by applying pressure only to the site where percutaneous insertion of an instrument occurred by using an inflatable balloon to apply the pressure to the puncture site. For example, the hemostatic device TR Band™ (US2009/0138039, US2013/0116725, US2013/0178894, US2013/0282048, EP1382306, JP2007075650, JP2005318998, JP20040415990) from Terumo Kabushiki Kaisha, JP, uses a single inflatable balloon to be positioned at the puncture site and is configured to apply pressure to the puncture site to stop bleeding. Ward (US2010/179586) describes a device for controlling bleeding from blood vessels located in difficult-to-compress regions of the body (especially the abdomen, pelvic or groin region) by applying intense, direct pressure.

Radial artery occlusion (RAO) is a common complication post trans-radial access (TRA) catheterization, affecting up to ten percent of the patients. As most parts of the hands have a dual blood supply through the ulnar and radial artery, RAO remains undetected for various reasons. First, the ulnar artery provides collateral feeding to the affected part of the hand, thereby making RAO more difficult to detect. In addition, patients are often not checked for the patency of the radial artery post TRA intervention. RAO not only poses ischemic complication to the hand, if blood flow through the ulnar artery is significantly diminished or blocked, but also prevents any future interventions through TRA. Undetected RAO may also render the ipsilateral ulnar artery unusable for instrumenting and cannulating the ulnar artery, which is the last remaining major artery supplying blood to the hand, as any compromise in the ulnar artery patency can expose the patient's hand at further risk of ischemia.

To prevent RAO, a delicate balance needs to be achieved between stopping the bleeding at the vascular access site and simultaneously allowing blood to flow through the radial artery, what is described as "patent hemostasis." In addition to RAO, after TRA intervention the radial artery caliber reduces, likely due to intimal hyperplasia, and such vascular changes can potentially interfere with any future intervention through the same vascular access. While conventional hemostatic devices stop the bleeding, these devices are not adapted to ensure that the necessary blood flow through the radial artery is maintained, thereby leading to RAO or affecting radial artery caliber.

It would therefore be desirable to provide a system for a vascular hemostatic system aimed at maintaining patent hemostasis by compressing radial artery adequately enough to stop bleeding, while still allowing blood flow through the artery at the same time. Such mechanism will significantly reduce RAO or reduction in caliber of the vessel and eventually ischemic complications to the hand.

### SUMMARY OF THE INVENTION

The present invention overcomes the aforementioned drawbacks by providing a system for applying occlusive pressure to the ulnar and radial artery using a vascular hemostatic device capable of simultaneously or independently compressing the ulnar and radial arteries. The vascular hemostatic device creates sufficient pressure on the radial and ulnar artery, but not the tissue in-between, resulting in patent hemostasis.

It is an aspect of the invention to provide a hemostatic device including a flexible band, which includes an inner surface, for enclosure about the wrist at a puncture site. A first pressure device and a second pressure device are coupled to the inner surface of the flexible band. The second pressure device is positioned relative to the first pressure device such that a distance between the first pressure device and the second pressure device results in the first pressure device being positioned proximal a first artery and the second pressure device being positioned proximal a second artery when the flexible band is enclosed about the wrist. For example, the pressure device can be a pressure pad wherein the pressure is adjustable, for example, with means of a screw or a lever, or the pressure device could be a balloon, wherein the pressure is adjustable, for example, by injection or removal of air, a liquid, or a gelatinous matrix. As one example, upon inflation of the first balloon and the second balloon, patent hemostasis can be achieved in the first artery while occlusive pressure is generated on the second artery. By adjusting pressure in the first balloon, the second balloon, or both, patent hemostasis can therefore be achieved. As one example, the first artery may be the radial artery and the second artery may be the ulnar artery. As another example, the first artery may be the ulnar artery and the second artery may be the radial artery.

The foregoing and other aspects and advantages of the invention will appear from the following description. In the description, reference is made to the accompanying drawings, which form a part hereof, and in which there is shown by way of illustration a preferred embodiment of the invention. Such embodiment does not necessarily represent the full scope of the invention, however, and reference is made therefore to the claims and herein for interpreting the scope of the invention.

### BRIEF DESCRIPTION OF THE DRAWINGS

FIG. 1 is a bottom view of an exemplary hemostatic device according to the present invention; and
FIG. 2 is a cross sectional view of the exemplary hemostatic device of FIG. 1 applied to a patient's wrist during use.

### DETAILED DESCRIPTION OF THE INVENTION

The invention is defined in claims 1, 2. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

Described here are systems for achieving patent hemostasis in the arteries of a patient's wrist following an interventional procedure, such as an angioplasty. In general, the system includes a flexible band to which two pressure devices (e.g., pressure pads or balloons) are coupled. The flexible band can be comprised of one or more materials resulting in portions of the flexible band having different flexibility, stiffness, or both. As one example, the portion of the flexible band to which the balloons are coupled could have less flexibility or higher stiffness to enhance directional pressure of the balloons towards the ulnar and/or radial artery. Also, the portion of the flexible band to which the balloons are coupled could have less flexibility or higher stiffness to reduce pressure to the wrist's tissue adjacent to the balloons. The balloons are positioned relative to each other such that when the flexible band is enclosed around the patient's wrist, the first balloon is positioned proximal to the radial artery and the second balloon is positioned proximal to the ulnar artery. The pressure in the balloons can be controlled such that occlusive pressure can be achieved in the ulnar artery while maintaining non-occlusive pressure in the radial artery, thereby achieving patent hemostasis in the radial artery.

Currently available hemostatic devices utilize a single pressure device, such as a pressure pad or balloon to achieve occlusive pressure following an interventional procedure. As one example, the pressure in this balloon is reduced in incremental steps and at timed intervals following a procedure. At each reduced pressure level, if there is no bleeding present, then the reduced pressure will be maintained until the next pressure reduction. Otherwise, an increased pressure is maintained until the next time interval has passed. This process can be very time consuming, often requiring up to two hours and multiple checks on the patient and adjustments of the pressure supplied by the device.

The systems described here, however, overcome the drawbacks of these earlier devices by providing control over the pressures applied to both the radial and ulnar arteries in a patient's wrist. With this dual control, patent hemostasis can be achieved in the radial artery while maintaining occlusive pressure in the ulnar artery. The advantage of this setup is that these pressures can be maintained until bleeding has stopped, which can be achieved without the frequent checkups and pressure changes require by currently available devices.

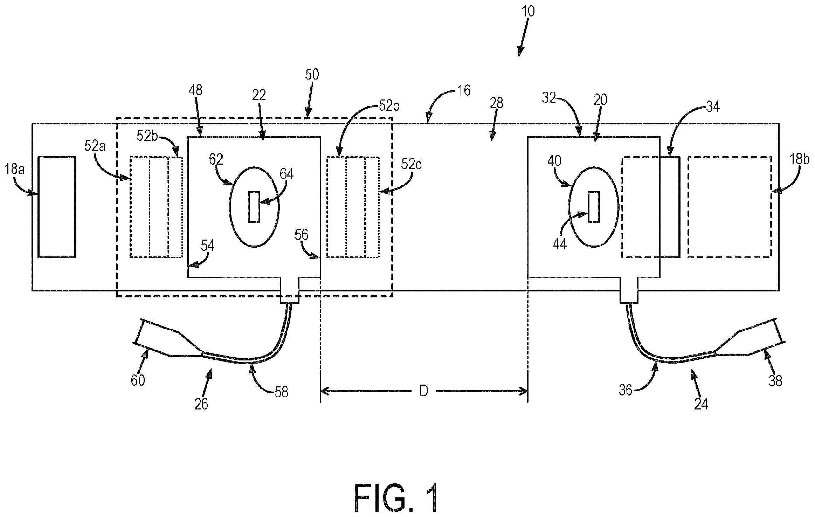

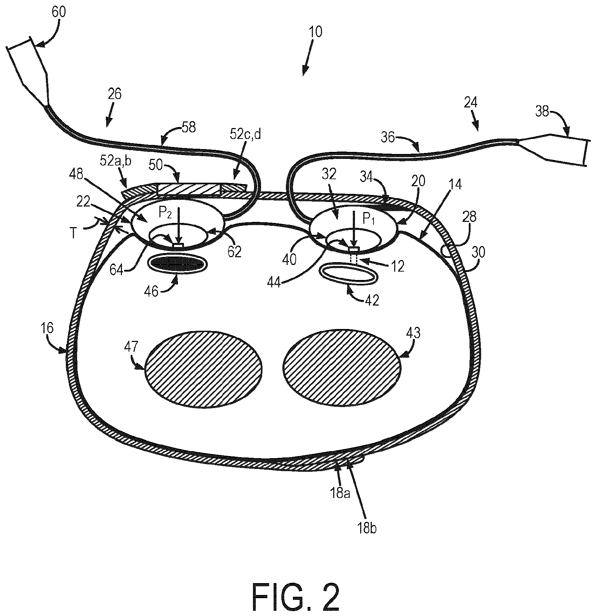

Referring now to FIGS. 1 and 2, an example hemostatic device 10 is shown. The hemostatic device 10 may be used to stop bleeding at a puncture site 12 following the removal of an instrument, such as a catheter, that was inserted percutaneously into an artery through a puncture formed on a wrist 14 of a patient. As one example, the hemostatic device may be used to stop bleeding at a puncture site that is formed following a medical procedure, such as an angioplasty. Generally, the hemostatic device 10 includes a flexible band 16 configured to be wrapped around the wrist 14 of a patient. The hemostatic device 10 also generally includes band fasteners 18a, 18b for securing the flexible band 16 to the wrist 14, a first balloon 20 and a second balloon 22 coupled to a first inflator 24 and a second inflator 26, respectively.

In general, the hemostatic device 10 is configured such that the first balloon 20 and the second balloon 22 are coupled to the flexible band 16 and spaced apart by a distance, D. This distance is selected such that when the flexible band 16 is enclosed around a patient's wrist 14, the first balloon 20 will be positioned proximal to a first artery and the second balloon 22 will be positioned proximal to a second artery. As an example, the first and second arteries can include the radial and ulnar arteries.

Referring again to FIGS. 1 and 2, the flexible band 16 may be constructed from a flexible material used in medical devices, such as, but not limited to, polymers, polymer blends, and thermoplastics. In some instances, the flexible material can include one or more materials, which can result in portions of the flexible band 16 having different flexibility, stiffness, or both. As one example, the portion of the flexible band 16 to which the first balloon 20 and the second balloon 22 are coupled could have less flexibility, higher stiffness, or both, to enhance directional pressure of the first and second balloons 20, 22 towards the radial artery 42 and/or ulnar artery 47, and/or to reduce pressure to the wrist's 14 tissue adjacent to the first balloon 20 and second balloon 22.The flexible band 16 can be coupled to the wrist 14 of a patient by being wrapped around an outside surface of the wrist 14. Preferably, the flexible material can also be bio-compatible. In some embodiments, the flexible band 16 can be constructed from a flexible material that is also transparent, which may provide a user visualization of the puncture site 12 where bleeding is to be stopped. The material used in the construction of the flexible band 16 may be in the form of a sheet having a thickness, T, that is sufficient to wrap the flexible band 16 around the wrist 14. In one example, the thickness, T, of the flexible band 16 can be between about 0.1 mm and about 5.0 mm; however, it will be appreciated that thinner or thicker materials that are sufficiently strong and flexible may also be utilized.

The band fasteners 18a, 18b may be positioned on opposing ends of the flexible band 16, as shown in FIG. 1. In one example, band fastener 18a is coupled to an inner surface 28 of the flexible band 16 and band fastener 18b is coupled to an outer surface 30 of the flexible band 16. Thus, when the flexible band 16 is wrapped around the wrist 14, the band fasteners 18a, 18b may overlap and secure to one another, as shown in FIG. 2. In one non-limiting example, the band fasteners 18a, 18b may be Velcro® to secure the flexible band 16 to the wrist 14. Other suitable securing mechanisms that could be used to fasten the flexible band 16 include, but are not limited to, snaps, buttons, clips, and members such as ratcheting clasps and other buckles through which the ends of the flexible band 16 can be passed.

The first balloon 20 may be positioned on the inner surface 28 of the flexible band 16 adjacent the band fastener 18b, as shown in FIG. 1. The first balloon 20 may be constructed of a flexible material that is inflatable upon introduction of a fluid (e.g., air, gas, liquid, etc.) into the first balloon 20. When the first balloon is so inflated, it applies a pressure to the puncture site 12 on the wrist 14. In some embodiments, the first balloon 20 is composed of a material that allows the puncture site 12 to be observable. For example, a material similar to that making up the flexible band 16 may be used. In one embodiment, the first balloon 20 may be transparent to ensure that the puncture site 12 is externally visible.

The first balloon 20 may be in the form of one or more sheets, for example, that are sealed together by any suitable process, such as adhesion, to form a first cavity 32, as shown in FIG. 2. In this configuration, the sheets forming the first balloon 20 may be of any suitable thickness. As one example, such sheets can be substantially square in a deflated state, as shown in FIG. 1. As another example, however, the sheets can be rectangular or any other suitable shape (e.g., round or oval) when in the deflated state.

The first balloon 20 may be coupled to the flexible band 16 by a first connector 34 provided on the inner surface 28 of the flexible band 16. The first connector 34 may be a fixed connector, such that the first balloon 20 remains stationary relative to movement of the second balloon 22. In one example, the first connector 34 may secure the first balloon 20 to the inner surface 28 of the flexible band 16 via any suitable adhesion technique. As one example, the first connector 34 may include welding the first balloon 20 to the inner surface 28 of the flexible band 16. Other suitable securing mechanisms include, but are not limited to Velcro®, snaps, buttons, clips, and the like.

As shown in FIGS. 1 and 2, the first balloon 20 is connected to the first inflator 24 for introducing a fluid and/or gas into the first cavity 32 of the first balloon 20. The first inflator 24 may include a first connector tube 36 coupled to the first cavity 32 at one end, and a first valve 38 at an opposing end of the first connector tube 36. As one example, the first valve 38 can be a one-way check valve. Inflation of the first balloon 20 may be achieved by inserting the protruding tip of a syringe (not shown) into the first connector tube 36 and pushing a plunger on the syringe so as to introduce fluid and/or gas within the syringe through the first inflator 24 into the first balloon 20. Once fluid and/or gas has been injected into the first balloon 20 and the protruding tip of the syringe has been withdrawn from the first inflator 24, the first valve 38 may be closed to inhibit fluid and/or gas from leaking out, thereby maintaining the first balloon 20 in an inflated state.

In some embodiments, the first balloon 20 may include a first bladder 40 positioned in the first cavity 32. The first bladder 40 may be liquid-filled, for example. As in other embodiments, inflation of the first cavity 32 may result in occlusive pressure applied to the radial artery 42, as will be described in further detail below. Upon inflation of the first cavity 32, the first bladder 40 may be positioned or otherwise located proximal to a radial artery 42 that is positioned above a radius bone 43 of the wrist 14. As will be described below, the first bladder 40 can be configured to allow visual inspection of the patient's pulse. For example, the first bladder 40 can be filled with a liquid or gel having a viscosity that is suitable to indicate pulsations caused by the patient's pulse. In some embodiments, the liquid or gel used to fill the first bladder 40 can be colored to provide additional visualization of the patient's pulse. In this configuration, the hemostatic device 10 thus includes a simple visual indication of whether there is pulsatile flow passing through the radial artery 42. Absence of a visual indication of a pulse in the first bladder 40 can thus indicate occlusion in the radial artery 42.

To help align the first bladder 40 with the radial artery 42, a first marker 44 may be provided on the first balloon 20 for positioning the first balloon 20 at the puncture site 12 where bleeding is to be stopped (i.e., the radial artery 42). In one non-limiting example, the first marker 44 may be characterized by a color that enables the first balloon 20 to be properly positioned at the puncture site 12.

Similar to the first balloon 20, the second balloon 22 may be positioned on the inner surface 28 of the flexible band 16 adjacent the band fastener 18a, as shown in FIG. 1. The second balloon 22 may be constructed of a flexible material that is inflatable upon a fluid (e.g., air, gas, liquid, etc.) being introduced therein, thereby applying pressing to an ulnar artery 46 in the wrist 14 (see FIG. 2). In some embodiments, the material making up the second balloon 22 may be any suitable material that allows visual inspection of the alignment of the second balloon 22 with the ulnar artery 46. For example, a material similar to that making up the flexible band 16, the first balloon 20, or both may be used. For example, in one embodiment, the second balloon 22 may be transparent to allow for the second balloon 22 to be properly aligned with the ulnar artery 46.

In some embodiments, the second balloon 22 may be in the form of one or more sheets that are sealed together by any suitable process, such as adhesion, to form a second cavity 48, as shown in FIG. 2. The sheets forming the second balloon 22 may be of any suitable thickness. As one example, such sheets can be substantially square when in a deflated state, as shown in FIG. 1. As another example, however, the sheets can be rectangular or any other suitable shape (e.g., round or oval) when in a deflated state.

The second balloon 22 may be coupled to a second connector 50 provided on the outer surface 30 of the flexible band 16. The second connector 50 may be an adjustable connector configured to engage a plurality of fasteners 52a, 52b, 52c, 52d. In one example, the plurality of fasteners 52a, 52b, 52c, 52d can be provided on the outer surface 30 of the flexible band 16 in order to connect the second balloon 22 to the flexible band 16. In the example shown in FIG. 1, the plurality of fasteners 52a, 52b are positioned adjacent a first edge 54 of the second balloon 22 nearest the band fastener 18a. The remaining plurality of fasteners 52b, 52c may be positioned on an opposing, second edge 56 of the second balloon 22, as shown in FIG. 1. Although the plurality of fasteners 52a, 52b, 52c, 52d include four fasteners, it is contemplated that a lesser or greater quantity of fasteners may be provided to sufficiently couple the second balloon 22 to the flexible band 16. In one example, the second connector 50 may secure the second balloon 22 to the inner surface 28 of the flexible band 16 via any suitable releasable adhesion technique. Other suitable securing mechanisms include, but are not limited to Velcro®, snaps, buttons, clips, and the like.

By utilizing an adjustable connector for the second connector 50, the second balloon 22 may be translated along, and re-attached to, the flexible band 16 such that a distance, D, between the first balloon 20 and the second balloon 22 may be adjusted. Advantageously, this configuration of the hemostatic device 10 allows the device to be used on various sizes and shapes of wrists 14, and to accommodate varying locations of the radial artery 42 and the ulnar artery 46 to which the first balloon 20 and the second balloon 22, respectively, should align.

As shown in FIGS. 1 and 2, the second balloon 22 is connected to the second inflator 26 for introducing a fluid and/or gas into the second cavity 48 of the second balloon 22. The second inflator 26 may include a second connector tube 58 coupled to the second cavity 48 at one end, and a second valve 60 at an opposing end of the second connector tube 58. As one example, the second valve 60 can be a one-way check valve. Inflation of the second balloon 22 may be achieved by inserting the protruding tip of a syringe (not shown) into the second connector tube 58 and pushing a plunger on the syringe so as to introduce fluid and/or gas within the syringe through the second inflator 26 into the second balloon 22. Once fluid and/or gas has been injected into the second balloon 22 and the protruding tip of the syringe has been withdrawn from the second inflator 26, the second valve 60may be closed to inhibit fluid and/or gas from leaking out, thereby maintaining the second balloon 22 in an inflated state.

In some embodiments, the second balloon 22 may include a second bladder 62 positioned in the second cavity 48. The second bladder 62 may be liquid-filled, for example. As in other embodiments, inflation of the second cavity 48 may result in occlusive pressure applied to the ulnar artery 46, as will be described in further detail below. Upon inflation of the second cavity 48, the second bladder 62 may be proximal to the ulnar artery 46 that is positioned above an ulnar bone 47 of the wrist 14. As will be described below, the second bladder 62 can be configured to allow visual inspection of the patient's pulse. For example, the second bladder 62 can be filled with a liquid or gel having a viscosity that is suitable to indicate pulsations caused by the patient's pulse. In some embodiments, the liquid or gel used to fill the second bladder 62 can be colored to provide additional visualization of the patient's pulse. In this configuration, the hemostatic device 10 thus includes a simple visual indication of whether there is pulsatile flow passing through the ulnar artery 46. Absence of a visual indication of a pulse in the second bladder 62 can thus indicate occlusion in the ulnar artery 46.

To help align the second bladder 62 with the ulnar artery 46, a second marker 64 may be provided for positioning the second balloon 22 at the ulnar artery 46 where intra-arterial blood flow is to be stopped. In one non-limiting example, the second marker 64 may be characterized by a color that enables the second balloon 22 to be properly positioned and aligned with the ulnar artery 46.

Having generally described several different embodiments of a hemostatic device for achieving patent hemostasis, an example of a method for using such a hemostatic device 10 to achieve patent hemostasis is now described. Once the interventional procedure (e.g., angioplasty) is completed and the sheath (not shown) is removed, the hemostatic device 10 is attached to the patient's wrist 14. To attach the hemostatic device 10 to a patient's wrist 14, the first balloon 20 and the second balloon 22 are placed in a deflated state over the radial artery 42 and ulnar artery 46, respectively. A user may then wrap the flexible band 16 around the wrist 14, and secure the flexible band 16 near both ends thereof with the band fasteners 18a, 18b. Once the hemostatic device 10 is securely attached to the patient's wrist 14, the first balloon 20 covering the radial artery 42 may be filled with air, for example, to apply a first occlusive pressure P₁ (see FIG. 2) to the radial artery 42, thereby stopping both bleeding and intra-arterial blood-flow at the puncture site 12. Then, similarly, the second balloon 22 covering the ulnar artery 46 may be filled with air to apply a second occlusive pressure P₂ to the ulnar artery 46, thereby stopping intra-arterial blood flow. As a result, there will be significantly limited blood supply to the hand as occlusive pressure (i.e., P₁ and P₂) on both the radial artery 42 and the ulnar artery 46 has been applied.

Once the first balloon 20 and the second balloon 22 are filled to the desired pressure to achieve occlusion of the radial artery 42 and ulnar artery 46, respectively, the corresponding valves 38, 60 may be closed to inhibit leakage of air from the first cavity 32 and/or the second cavity 48. Thus, the first balloon 20 and the second balloon 22 will maintain compression against the radial artery 42 and the ulnar artery 46 (see FIG. 2), respectively.

Subsequently, pressure P₁ may be released from the first balloon 20 covering the radial artery 42 until a blood spurt from the puncture site 12 of the radial artery 42 underneath the first balloon 20 is seen. In this manner, the amount of pressure required to completely occlude the radial artery 42 is demonstrated, and the spurt of blood that is flowing ante-gradely will flush out any thrombus present at the site of sheath insertion. Once blood flow has been observed, the pressure P₁ in the first balloon 20 may be increased to stop any further bleeding by introducing additional air (e.g., a few cc) through the first inflator 24 into the first balloon 20 covering the punctured, radial artery 42.

At this point, blood supply to the hand is maintained only through the radial artery 42, as the adequate pressure P₂ on the ulnar artery 46 has been maintained to completely stop the ante-grade flow. In one example, the transition from a pale hand (i.e., lack of blood supply) at the time of occlusion of both the radial artery 42 and the ulnar artery 46 changing to red demonstrates that the blood supply to the hand has been restored by reducing the pressure P₁ on the radial artery 42. In alternative embodiments, pulse oximetry on the fingers or thumb can be used to provide appropriate tracking of the blood supply. Similarly, observing the color in the palm of the hand can indirectly demonstrate maintained blood supply to the hand.

The pressure P₁ from the first balloon 20 covering the radial artery 42 may be gradually released, thereby removing the pressure P₁. As one example, the pressure P₁ can be gradually released in about an hour. The timing for complete removal of the pressure P₁ depends on several factors. Some example factors include, but are not limited to, how much heparin is given to the patient during the procedure and the systemic blood pressure. Once it is confirmed that good hemostasis has been achieved in the radial artery 42, the pressure P₂ from the second balloon 22 covering the ulnar artery 46 can be removed. At this point, secured hemostasis will have been achieved and patent and ante-grade flow through the radial artery 42 will have been maintained.

In one non-limiting example, the first balloon 20 and the second balloon 22 may incorporate the first bladder 40 and the second bladder 62, respectively. The first bladder 40 and the second bladder 62, as previously described, may be liquid-filled bladders on the side proximal to the radial artery 42 and the ulnar artery 46. Once the pressure P₁, P₂ in the balloon 20,22 reaches a predetermined pressure, but before the artery 42, 46 is occluded, the liquid-filled bladder 40, 62 may provide visual inspection of the pulse. Occlusion of the artery 42, 46 may be indicated by the absence of a pulse. Additionally or alternatively, the liquid within the bladder 40, 62 may be colorful for better observation of the pulse. In yet another example, the liquid may be a gel characterized by a viscosity allowing visualization of the pulse.

The present invention has been described in terms of one or more preferred embodiments, and it should be appreciated that many equivalents, alternatives, variations, and modifications, aside from those expressly stated, are possible and within the scope of the invention.

## Claims

1. A hemostatic device (10) comprising:
a flexible band (16) for enclosure about a wrist (14) at a puncture site (12), the flexible band (16) including an inner surface (28);
a first balloon (20) coupled to the inner surface (28) of the flexible band (16); and
a second balloon (22) coupled to the inner surface (28) of the flexible band (16) and positioned relative to the first balloon (20) such that a distance between the first balloon (20) and second balloon (22) results in the first balloon (20) being positioned proximal an ulnar artery (46) and the second balloon (22) being positioned proximal a radial artery (42) when the flexible band (16) is enclosed about the wrist (14);
wherein, upon inflation of the first balloon (20) and the second balloon (22), patent hemostasis is achieved on the ulnar artery (46) and occlusive pressure is generated on the radial artery (42).

2. A hemostatic device (10) comprising:
a flexible band (16) for enclosure about a wrist (14) at a puncture site (12), the flexible band (16) including an inner surface (28);
a first balloon (20) coupled to the inner surface (28) of the flexible band (16); and
a second balloon (22) coupled to the inner surface (28) of the flexible band (16) and positioned relative to the first balloon (20) such that a distance between the first balloon (20) and second balloon (22) results in the first balloon (20) being positioned proximal an radial artery (42) and the second balloon (22) being positioned proximal an ulnar artery (46) when the flexible band (16) is enclosed about the wrist (14);
wherein, upon inflation of the first balloon (20) and the second balloon (22), patent hemostasis is achieved on the radial artery (42) and occlusive pressure is generated on the ulnar artery (46).

3. The hemostatic device as recited in any of claims 1 or 2, wherein at least one of the first balloon (20) and the second balloon (22) is coupled to the flexible band (16) by an adjustable connector (50);
wherein, upon adjustment of the adjustable connector (50) along the flexible band (16), the distance between the first balloon (20) and the second balloon (22) is changed.

4. The hemostatic device as recited in claim 3 wherein the adjustable connector (50) is configured to engage a plurality of fasteners (52a, 52b, 52c, 52d) to secure at least one of the first balloon (20) or the second balloon (22) to the flexible band (16).

5. The hemostatic device as recited in any of claims 1 or 2, wherein at least one of the first balloon (20) or the second balloon (22) is coupled to the flexible band (16) by a fixed connector (34).

6. The hemostatic device as recited in any of claims 1 or 2, wherein at least one of the first balloon (20) or the second balloon (22) includes a liquid-filled bladder (40, 62) that is positioned proximal to at least one of the radial artery (42) or the ulnar artery (46) when the flexible band (16) is enclosed about the wrist (14).

7. The hemostatic device as recited in any of claims 1 or 2, wherein at least one of the first balloon (20) or the second balloon (22) includes a marker (44, 64) for positioning the at least one of the first balloon (20) or the second balloon (22) at a site on the wrist (14) where at least one of bleeding or intra-arterial blood flow is to be stopped.

8. The hemostatic device as recited in claim 7 wherein the marker (44, 64) is **characterized by** a color to enable the at least one of the first balloon (20) or the second balloon (22) to be positioned at the site on the wrist (14) where the at least one of bleeding and intra-arterial blood flow is to be stopped.

9. The hemostatic device as recited in any of claims 1 or 2, wherein, upon inflation of the first balloon (20) and the second balloon (22), a first occlusive pressure is generated by the first balloon (20) sufficient to stop both bleeding and intra-arterial blood-flow at the radial artery (42), and a second occlusive pressure is generated by the second balloon (22) sufficient to stop intra-arterial blood-flow at the ulnar artery (46).

10. The hemostatic device as recited in any of claims 1 or 2, wherein patent hemostasis is achieved in the radial artery (42) by inflating the first balloon (20) to a non-occlusive pressure.

11. The hemostatic device as recited in any of claims 1 or 2, wherein at least one of the flexible band (16), the first balloon (20), or the second balloon (22) is constructed of a transparent material for visualization of a site on the wrist (14) where at least one of bleeding or intra-arterial blood flow is to be stopped.

12. The hemostatic device as recited in any of claims 1 or 2, further comprising at least one inflator (24, 26) coupled to at least one of the first balloon (20) or the second balloon (22) for inflating the at least one of the first balloon (20) or the second balloon (22).

13. The hemostatic device as recited in claim 12 wherein the at least one inflator (24, 26) includes a valve (38, 60) configured to close so as to maintain a desired level of pressure in the at least one of the first balloon (20) or the second balloon (22).

## Patentansprüche

1. Hämostatische Vorrichtung (10), umfassend:
ein flexibles Band (16) zum Umschließen um ein Handgelenk (14) an einer Punktionsstelle (12), wobei das flexible Band (16) eine Innenfläche (28) aufweist;
einen ersten Ballon (20), der mit der Innenfläche (28) des flexiblen Bandes (16) verbunden ist; und
einen zweiten Ballon (22), der mit der Innenfläche (28) des flexiblen Bandes (16) gekoppelt und relativ zum ersten Ballon (20) so positioniert ist, dass ein Abstand zwischen dem ersten Ballon (20) und dem zweiten Ballon (22) dazu führt, dass der erste Ballon (20) proximal einer ulnaren Arterie (46) und der zweite Ballon (22) proximal einer radialen Arterie (42) positioniert ist, wenn das flexible Band (16) um das Handgelenk (14) herum umschlossen ist;
wobei beim Aufblasen des ersten Ballons (20) und des zweiten Ballons (22) eine offenliegende Hämostase an der ulnaren Arterie (46) erreicht wird und ein Verschlussdruck an der radialen Arterie (42) erzeugt wird.

2. Hämostatische Vorrichtung (10), umfassend:
ein flexibles Band (16) zum Umschließen um ein Handgelenk (14) an einer Punktionsstelle (12), wobei das flexible Band (16) eine Innenfläche (28) aufweist;
einen ersten Ballon (20), der mit der Innenfläche (28) des flexiblen Bandes (16) verbunden ist; und
einen zweiten Ballon (22), der mit der Innenfläche (28) des flexiblen Bandes (16) gekoppelt und relativ zum ersten Ballon (20) so positioniert ist, dass ein Abstand zwischen dem ersten Ballon (20) und dem zweiten Ballon (22) dazu führt, dass der erste Ballon (20) proximal einer radialen Arterie (42) und der zweite Ballon (22) proximal einer ulnaren Arterie (46) positioniert ist, wenn das flexible Band (16) um das Handgelenk (14) herum umschlossen ist;
wobei beim Aufblasen des ersten Ballons (20) und des zweiten Ballons (22) eine offenliegende Hämostase an der radialen Arterie (42) erreicht wird und ein Verschlussdruck an der ulnaren Arterie (46) erzeugt wird.

3. Die hämostatische Vorrichtung nach einem der Ansprüche 1 oder 2, wobei mindestens einer von dem ersten Ballon (20) und dem zweiten Ballon (22) mit dem flexiblen Band (16) durch einen verstellbaren Verbinder (50) verbunden ist;
wobei bei Einstellung des einstellbaren Verbinders (50) entlang des flexiblen Bandes (16) der Abstand zwischen dem ersten Ballon (20) und dem zweiten Ballon (22) verändert wird.

4. Die hämostatische Vorrichtung nach Anspruch 3, wobei der einstellbare Verbinder (50) so konfiguriert ist, dass er mit einer Vielzahl von Befestigungselementen (52a, 52b, 52c, 52d) in Eingriff kommt, um mindestens einen des ersten Ballons (20) oder des zweiten Ballons (22) an dem flexiblen Band (16) zu befestigen.

5. Die hämostatische Vorrichtung nach einem der Ansprüche 1 oder 2, wobei mindestens einer aus dem ersten Ballon (20) oder dem zweiten Ballon (22) durch einen festen Verbinder (34) mit dem flexiblen Band (16) verbunden ist.

6. Die hämostatische Vorrichtung nach einem der Ansprüche 1 oder 2, wobei mindestens einer aus dem ersten Ballon (20) oder dem zweiten Ballon (22) eine flüssigkeitsgefüllte Blase (40, 62) aufweist, die proximal zu mindestens einer der radialen Arterie (42) oder der ulnaren Arterie (46) positioniert ist, wenn das flexible Band (16) um das Handgelenk (14) umschlossen ist.

7. Die hämostatische Vorrichtung nach einem der Ansprüche 1 oder 2, wobei mindestens einer des ersten Ballons (20) oder des zweiten Ballons (22) eine Markierung (44, 64) zum Positionieren des mindestens einen des ersten Ballons (20) oder des zweiten Ballons (22) an einer Stelle am Handgelenk (14) aufweist, an der mindestens eine Blutung oder ein intraarterieller Blutfluss gestoppt werden soll.

8. Die Hämostatische Vorrichtung nach Anspruch 7, bei der die Markierung (44, 64) durch eine Farbe gekennzeichnet ist, die es ermöglicht, den ersten Ballon (20) und/oder den zweiten Ballon (22) an der Stelle am Handgelenk (14) zu positionieren, an der die Blutung und/oder der intraarterielle Blutfluss gestoppt werden soll.

9. Die hämostatische Vorrichtung nach einem der Ansprüche 1 oder 2, wobei beim Aufblasen des ersten Ballons (20) und des zweiten Ballons (22) durch den ersten Ballon (20) ein erster Verschlussdruck erzeugt wird, der ausreicht, um sowohl die Blutung als auch den intraarteriellen Blutfluss an der radialen Arterie (42) zu stoppen, und durch den zweiten Ballon (22) ein zweiter Verschlussdruck erzeugt wird, der ausreicht, um den intraarteriellen Blutfluss an der ulnaren Arterie (46) zu stoppen.

10. Die hämostatische Vorrichtung gemäß einem der Ansprüche 1 oder 2, wobei die offenliegende Hämostase in der radialen Arterie (42) durch Aufblasen des ersten Ballons (20) auf einen nicht okklusiven Druck erreicht wird.

11. Die hämostatische Vorrichtung nach einem der Ansprüche 1 oder 2, wobei mindestens einer der flexiblen Bänder (16), der erste Ballon (20) oder der zweite Ballon (22) aus einem transparenten Material zur Sichtbarmachung einer Stelle am Handgelenk (14) hergestellt ist, an der mindestens eine Blutung oder ein intraarterieller Blutfluss gestoppt werden soll.

12. Die hämostatische Vorrichtung nach einem der Ansprüche 1 oder 2, die ferner mindestens eine Aufblasvorrichtung (24, 26) aufweist, die mit mindestens einem des ersten Ballons (20) oder des zweiten Ballons (22) zum Aufblasen des mindestens einen des ersten Ballons (20) oder des zweiten Ballons (22) gekoppelt ist.

13. Die hämostatische Vorrichtung nach Anspruch 12, wobei die mindestens eine Aufblasvorrichtung (24, 26) ein Ventil (38, 60) aufweist, das so konfiguriert ist, dass es sich schließt, um ein gewünschtes Druckniveau in dem mindestens einen des ersten Ballons (20) oder des zweiten Ballons (22) aufrechtzuerhalten.

## Revendications

1. Dispositif hémostatique (10) comprenant :
une bande flexible (16) destinée à entourer un poignet (14) au niveau d'un site de ponction (12), la bande flexible (16) comportant une surface intérieure (28) ;
un premier ballonnet (20) couplé à la surface intérieure (28) de la bande flexible (16) ; et
un second ballonnet (22) couplé à la surface intérieure (28) de la bande flexible (16) et positionné par rapport au premier ballonnet (20) de sorte qu'une distance entre le premier ballonnet (20) et le second ballonnet (22) ait pour effet que le premier ballonnet (20) soit positionné à proximité d'une artère ulnaire (46) et le second ballonnet (22) soit positionné à proximité d'une artère radiale (42) lorsque la bande flexible (16) entoure le poignet (14) ;
dans lequel, lors du gonflement du premier ballonnet (20) et du second ballonnet (22), une hémostase patente est obtenue sur l'artère ulnaire (46) et une pression occlusive est générée sur l'artère radiale (42).

2. Dispositif hémostatique (10) comprenant :
une bande flexible (16) destinée à entourer un poignet (14) au niveau d'un site de ponction (12), la bande flexible (16) comportant une surface intérieure (28) ;
un premier ballonnet (20) couplé à la surface intérieure (28) de la bande flexible (16) ; et
un second ballonnet (22) couplé à la surface intérieure (28) de la bande flexible (16) et positionné par rapport au premier ballonnet (20) de sorte qu'une distance entre le premier ballonnet (20) et le second ballonnet (22) ait pour effet que le premier ballonnet (20) soit positionné à proximité d'une artère radiale (42) et le second ballonnet (22) soit positionné à proximité d'une artère ulnaire (46) lorsque la bande flexible (16) entoure le poignet (14) ;
dans lequel, lors du gonflement du premier ballonnet (20) et du second ballonnet (22), une hémostase patente est obtenue sur l'artère radiale (42) et une pression occlusive est générée sur l'artère ulnaire (46).

3. Dispositif hémostatique selon l'une quelconque des revendications 1 ou 2, dans lequel au moins l'un du premier ballonnet (20) et du second ballonnet (22) est couplé à la bande flexible (16) par un raccord ajustable (50) ;
dans lequel, lors de l'ajustement du raccord ajustable (50) le long de la bande flexible (16), la distance entre le premier ballonnet (20) et le second ballonnet (22) est modifiée.

4. Dispositif hémostatique selon la revendication 3 dans lequel le raccord ajustable (50) est configuré pour se mettre en prise avec une pluralité de dispositifs de fixation (52a, 52b, 52c, 52d) pour fixer au moins l'un du premier ballonnet (20) ou du second ballonnet (22) à la bande flexible (16).

5. Dispositif hémostatique selon l'une quelconque des revendications 1 ou 2, dans lequel au moins l'un du premier ballonnet (20) ou du second ballonnet (22) est couplé à la bande flexible (16) par un raccord fixe (34) .

6. Dispositif hémostatique selon l'une quelconque des revendications 1 ou 2, dans lequel au moins l'un du premier ballonnet (20) ou du second ballonnet (22) comporte une vessie remplie de liquide (40, 62) qui est positionnée à proximité d'au moins l'une de l'artère radiale (42) ou de l'artère ulnaire (46) lorsque la bande flexible (16) entoure le poignet (14).

7. Dispositif hémostatique selon l'une quelconque des revendications 1 ou 2, dans lequel au moins l'un du premier ballonnet (20) ou du second ballonnet (22) comporte un marqueur (44, 64) pour positionner l'au moins un du premier ballonnet (20) ou du second ballonnet (22) au niveau d'un site sur le poignet (14) où au moins l'un d'un flux sanguin hémorragique ou intra-artériel doit être stoppé.

8. Dispositif hémostatique selon la revendication 7 dans lequel le marqueur (44, 64) est **caractérisé par** une couleur pour permettre que l'au moins un du premier ballonnet (20) ou du second ballonnet (22) soit positionné au niveau du site sur le poignet (14) où l'au moins un d'un flux sanguin hémorragique ou intra-artériel doit être stoppé.

9. Dispositif hémostatique selon l'une quelconque des revendications 1 ou 2, dans lequel, lors du gonflement du premier ballonnet (20) et du second ballonnet (22) une première pression occlusive est générée par le premier ballonnet (20) suffisante pour stopper à la fois un flux sanguin hémorragique et intra-artériel au niveau de l'artère radiale (42), et une seconde pression occlusive est générée par le second ballonnet (22) suffisante pour stopper un flux sanguin intra-artériel au niveau de l'artère ulnaire (46).

10. Dispositif hémostatique selon l'une quelconque des revendications 1 ou 2, dans lequel une hémostase patente est obtenue dans l'artère ulnaire (42) par gonflement du premier ballonnet (20) à une pression non occlusive.

11. Dispositif hémostatique selon l'une quelconque des revendications 1 ou 2, dans lequel au moins l'un de la bande flexible (16), du premier ballonnet (20), ou du second ballonnet (22) est construit en un matériau transparent pour une visualisation d'un site sur le poignet (14) où au moins l'un d'un flux sanguin hémorragique ou intra-artériel doit être stoppé.

12. Dispositif hémostatique selon l'une quelconque des revendications 1 ou 2, comprenant en outre au moins un gonfleur (24, 26) couplé à au moins l'un du premier ballonnet (20) ou du second ballonnet (22) pour gonfler l'au moins un du premier ballonnet (20) ou du second ballonnet (22).

13. Dispositif hémostatique selon la revendication 12 dans lequel l'au moins un gonfleur (24, 26) comporte une soupape (38, 60) configurée pour fermer de manière à maintenir un niveau de pression souhaité dans l'au moins un du premier ballonnet (20) ou du second ballonnet (22) .
